# EUROPÄISCHE PATENTSCHRIFT

(11) **EP 2 486 001 B1**
(45) Veröffentlichungstag und Bekanntmachung des Hinweises auf die Patenterteilung: **08.03.2017**
(21) Anmeldenummer: 10765776.9
(22) Anmeldetag: 04.10.2010
(51) Int. Cl.: C07C 209/34, C07C 211/15

(54) **VERFAHREN ZUR HERSTELLUNG VON 2,2-DIFLUORETHYLAMIN DURCH HYDRIERUNG VON 1,1-DIFLUOR-2-NITROETHAN**
METHOD FOR PRODUCING 2,2-DIFLUOROETHYLAMINE BY HYDROGENATING 1,1-DIFLUORO-2-NITROETHANE
PROCÉDÉ DE PRODUCTION DE 2,2-DIFLUOROÉTHYLAMINE PAR HYDROGÉNATION DE 1,1-DIFLUORO-2-NITROÉTHANE

(30) Priorität: 06.10.2009 EP 09172271; 08.10.2009 US 249815 P
(43) Veröffentlichungstag der Anmeldung: 15.08.2012
(73) Patentinhaber: Bayer Intellectual Property GmbH, 40789 Monheim am Rhein (DE)
(72) Erfinder: ANTONS, Stefan, 51373 Leverkusen (DE); LUI, Norbert, 51519 Odenthal (DE); GERLACH, Arne, 40239 Düsseldorf (DE)
(74) Vertreter: BIP Patents
(86) Internationale Anmeldenummer: PCT/EP2010/064700
(87) Internationale Veröffentlichungsnummer: WO 2011/042376

(56) Entgegenhaltungen:
- EP-A1- 0 116 886
- KLUGER R ET AL: "Carboxylic acid participation in amide hydrolysis. Evidence that separation of a nonbonded complex can be rate determining", JOURNAL OF THE AMERICAN CHEMICAL SOCIETY, AMERICAN CHEMICAL SOCIETY, US, Bd. 104, Nr. 10, 1. Januar 1982 (1982-01-01), Seiten 2891-2897, XP002504652, ISSN: 0002-7863, DOI: DOI:10.1021/JA00374A032 in der Anmeldung erwähnt
- DONETTI A ET AL: "N-(fluoroethyl)(imidazolylphenyl)formamid ines. The issue of the active species of mifentidine", JOURNAL OF MEDICINAL CHEMISTRY, AMERICAN CHEMICAL SOCIETY, WASHINGTON, US, Bd. 32, Nr. 5, 1. Mai 1989 (1989-05-01), Seiten 957-961,1, XP002573225, ISSN: 0022-2623, DOI: DOI:10.1021/JM00125A006 in der Anmeldung erwähnt
- DICKEY ET AL: "Fluorinated Aminoanthraquinone dyes", INDUSTRIAL AND ENGINEERING CHEMISTRY PRODUCT RESEARCH AND DEVELOPMENT, AMERICAN CHEMICAL SOCIETY, EASTON, PA, US, Bd. 48, Nr. 2, 1. Januar 1956 (1956-01-01) , Seiten 209-213, XP002559402, ISSN: 0536-1079, DOI: DOI:10.1021/IE50554A018 in der Anmeldung erwähnt
- SWARTS F: "Über einige fluorhaltige Alkylamine", CHEMISCHES ZENTRALBLATT,, Bd. 75, Nr. (II), 1. Januar 1904 (1904-01-01), Seiten 944-945, XP009126801,
- ANDREW E. FEIRING: "Perfluoroalkylation of the 2-nitropropyl anion. Evidence for an SRN1 process", J. ORG. CHEM., Bd. 48, Nr. 3, 1982, Seiten 347-354, XP002632860, DOI: 10.1021/jo00151a014
- F.L.M. PATTISON ET AL: "Toxic Fluorine Compounds. VI. omega-Fluoroalkylamines", J.AM.CHEM.SOC., Bd. 78, Nr. 14, Juli 1956 (1956-07), Seiten 3487-3489, XP002632861,
- D.J. COOK ET AL: "The Preparation and Reactions of Some Fluorine-containing Nitro Compounds", J. AM. CHEM. SOC., Bd. 76, Nr. 1, Januar 1954 (1954-01), Seiten 83-87, XP002632862, DOI: 10.1021/ja01630a022
- SHAO Y M ET AL: "Design, synthesis, and evaluation of trifluoromethyl ketones as inhibitors of SARS-CoV 3CL protease", BIOORGANIC & MEDICINAL CHEMISTRY, PERGAMON, GB, Bd. 16, Nr. 8, 15. April 2008 (2008-04-15) , Seiten 4652-4660, XP022617914, ISSN: 0968-0896, DOI: DOI:10.1016/J.BMC.2008.02.040 [gefunden am 2008-02-15]
- RAM S ET AL: "A GENERAL PROCEDURE FOR MILD AND RAPID REDUCTION OF ALIPHATIC AND AROMATIC NITRO COMPOUNDS USING AMMONIUM FORMATE AS A CATALYTIC HYDROGEN TRANSFER AGENT", TETRAHEDRON LETTERS, ELSEVIER, AMSTERDAM, NL, Bd. 25, Nr. 32, 1. Januar 1984 (1984-01-01), Seiten 3415-3418, XP000644519, ISSN: 0040-4039, DOI: DOI:10.1016/S0040-4039(01)91034-2

## Beschreibung

Die vorliegende Erfindung betrifft ein Verfahren zur Herstellung von 2,2-Difluorethylamin und/oder seiner Säureadditionssalze, umfassend die katalytische Hydrierung von 1,1-Difluor-2-nitroethan.

2,2-Difluorethylamin ist eine wichtige Zwischenverbindung bei der Wirkstoffherstellung. Es sind verschiedene Herstellungsmethoden für 2,2-Difluorethylamin bekannt.

Donetti et al. (J. Med. Chem. 1989, 32, 957-961) beschreiben die Synthese von 2,2-DifluorethylaminHydrochlorid ausgehend von 2,2-Difluoracetamid. Hierbei wird mit einer Diboran-Lösung in Tetrahydrofuran (THF) das gewünschte Amin hergestellt. Die Ausbeute beträgt 48%.

Kluger et al. (JACS 1982, 104, 10, 2891-2897) beschreiben die Synthese von 2,2-Difluorethylamin ausgehend vom Amid mit Natriumboranat und Bortrifluoridetherat. Die Ausbeute beträgt 60%.

Auch Kollonitsch (US 4,030,994) beschreibt eine Synthese von 2,2-Difluorethylamin, nämlich die Umsetzung von Ethylamin mit Fluoroxytrifluoromethan in Fluorwasserstoff unter UV Bestrahlung.

Die Herstellung von 2,2-Difluorethylamin wird auch bei Dickey et al. (Ind. Eng. Chem. 1956, 209- 213) beschrieben. 2,2-Difluor-1-chlorethan wird dort mit 28 %-igem Ammoniumhydroxid, d.h. 28 %-iger wässriger Ammoniak-Lösung, in einem Autoklaven zur Reaktion gebracht. Das Amin wurde so in einer Ausbeute von 65 % gewonnen.

Alle diese Verfahren lassen sich nicht im wirtschaftlich sinnvollen großtechnischen (industriellen) Maßstab durchführen. Die geringe Ausbeute und der Einsatz von teuren und gefährlichen Chemikalien wie z.B. Natriumboranat/BF₃ oder Diboran verhindern, dass die Verfahren nach Donetti et al. und Kluger et al. für die großtechnische Herstellung von 2,2-Difluorethylamin geeignet sind. Das Verfahren nach Kollonitsch et al. verwendet gefährliche Chemikalien und es wird kein reines 2,2-Difluorethylamin erhalten. Das Verfahren nach Dickey et al. ist ebenfalls für den großtechnischen Einsatz ungeeignet bzw. unwirtschaftlich, denn es benötigt eine sehr lange Reaktionszeit bei einer eher geringen Ausbeute. Weiterhin ist die Reaktionsmischung stark korrodierend, so dass auch deshalb die Wirtschaftlichkeit in Frage gestellt ist.

Ausgehend von den bekannten Verfahren zur Herstellung von 2,2-Difluorethylamin stellt sich nun die Aufgabe, ein alternatives Verfahren zu finden, durch das 2,2-Difluorethylamin einfach und kostengünstig hergestellt werden kann. Unter kostengünstigen Verfahren werden solche Verfahren verstanden, die ohne großen finanziellen Aufwand durchzuführen sind, weil die Ausgangsstoffe beispielsweise ungefährlich sind und/oder das gewünschte 2,2-Difluorethylamin in einer ausreichend hohen Ausbeute und Reinheit erhalten wird. Gleichfalls ist es vorteilhaft, die entsprechenden 2,2-Difluorethylamin-Säureadditionssalze zu erhalten, denn diese lassen sich ohne weiteres zum 2,2-Difluorethylamin umsetzen.

1,1-Difluor-2-nitroethan ist als Ausgangsstoff leicht verfügbar, denn es kann in hoher Ausbeute und in guter Reinheit mit dem in DE-A1-3305201 beschriebenen Verfahren hergestellt werden. Demzufolge wäre es wünschenswert ein Verfahren zu finden, in dem 1,1-Difluor-2-nitroethan zum gewünschten Amin reduziert wird.

Knunyaants et al. ("Aliphatic Fluoro Nitro Compounds Communication 3", Russian Chemical Bulletin (1964), Seiten 1538-1541) beschreiben die katalytische Hydrierung von Alkoxytrifluornitropopanen über Raney-Nickel, einem festen Katalysator, der aus feinen Körnern einer Nickel-AluminiumLegierung besteht, in Gegenwart von gasförmigen Wasserstoff zu 1-(Alkoxymethyl)-2,2,2-trifluorethylamin. Speziell wurde die Hydrierung von 1-(Ethoxymethyl)-2,2,2-trifluorethylamin mit gasförmigen Wasserstoff über Raney-Nickel in einem Autoklaven bei 60 atm (ca. 60 bar) Wasserstoffdruck beschrieben. Nach Aufarbeitung wurde 1-(Ethoxymethyl)-2,2,2-trifluorethylamin als Hydrochlorid in einer Ausbeute von 77 % isoliert.

Knunyants et al. (in Izvestiya Akademii Nauk SSSR (1966), (2), Seiten 250-253 in russischer Sprache bzw. in Russian Chemical Bulletin (1966), "Aliphatic Fluoro Nitro Compounds Communication 4", Seiten 226-228 in englischer Sprache) beschreiben Methoden zur Hydrierung fluorierter Nitrokohlenwasserstoffe, insbesondere von 1,1,1-Trifluor-2-nitroethan unter Verwendung von metallischem Palladium oder Eisen in Salzsäure, mit dem Ziel, das entsprechende Amin zu erhalten. Während die Verwendung von metallischem Palladium bei der Reduktion von 1,1,1-Trifluor-2-nitroethan lediglich zum N-(2,2,2-Trifluorethyl)hydroxylamin führt, kann bei Verwendung von Eisen in Salzsäure das gewünschte 2,2,2-Trifluorethylamin Hydrochlorid mit 77 % Ausbeute erhalten werden. Hierbei werden 1,1,1-Trifluor-2-nitroethan zusammen mit Salzsäure und Eisenspänen bei 90 °C bis 95 °C erhitzt, anschließend mit Natriumhydroxid alkalisch gestellt und das Trifluorethylamin mit Wasserdampf herausdestilliert. Das Produkt wird als Hydrochlorid isoliert.

Knunyants et al. (1966) beschreiben jedoch nicht die katalytische Hydrierung von 1,1-Difluor-2-nitroethan. Es wurde festgestellt, dass die bei Knunyants et al. beschriebenen Reduktions- bzw. Hydrierungsmethoden sich nicht ohne weiteres auf die Reduktion von 1,1-Difluor-2-nitroethan übertragen lassen. So führte die Reduktion durch Hydrierung von 1,1-Difluor-2-nitroethan mit Eisen in Salzsäure unter den bei Knunyants beschriebenen Bedingungen zu keinem 2,2-Difluorethylamin.

EP 0 116 886 beschriebt ein Verfahren zur Herstellung von 2,2-Difluor-2-nitroethan, sowie dass ein solches Nitroethan durch katalytische Hydrierung der Nitrogruppe zum entsprechenden Amin reduziert werden kann.Es wird jedoch kein Katalysator offenbart.

Es wurde nun ein Verfahren zur katalytischen Hydrierung von 1,1-Difluor-2-nitroethan gefunden, durch das 2,2-Difluorethylamin bzw. ein entsprechendes Säureadditionssalz in guten Ausbeuten und hoher Reinheit herstellbar ist, so dass im Allgemeinen anschließend keine komplexen Aufreinigungen notwendig sind, und das zugleich einfach und kostengünstig ist.

Gegenstand der Erfindung ist somit ein Verfahren zur Herstellung von 2,2-Difluorethylamin und/oder seiner Säureadditionssalze, umfassend die katalytische Hydrierung von 1,1-Difluor-2-nitroethan. Das erfindungsgemäße Verfahren ist im nachfolgenden Schema beispielhaft dargestellt,

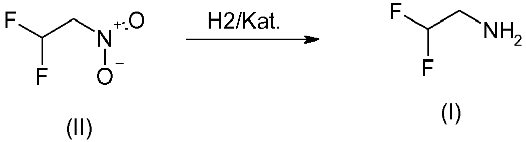

Die erfindungsgemäße katalytische Hydrierung findet in Gegenwart eines Katalysators statt, wobei der in der katalytischen Hydrierung eingesetzte Katalysator ein oder mehrere Metalle ausgewählt unter Palladium oder Platin enthält, wobei Raney-Nickel ausgeschlossen ist, wobei die katalytische Hydrierung in Gegenwart von anorganischer oder organischer Säure stattfindet und wobei der Katalysator PtO₂ oder Pd(OH)₂ auf Aktivkohle ist, und wobei gasförmiger Wasserstoff in das Reaktionsgefäß geleitet oder im Reaktionsgefäß *in situ* erzeugt wird.

Die Metalle können in geträgerter Form, d.h. auf einen beliebigen, vorzugsweise anorganischen, Träger aufgebracht, vorliegen. Als Träger eigenen sich beispielsweise Kohlenstoff (Kohle oder Aktivkohle), Aluminiumoxid, Siliciumdioxid, Zirkondioxid oder Titandioxid. Erfindungsgemäß bevorzugte Katalysatoren enthalten ein oder mehrere Metalle der Gruppen 8 - 10 des Periodensystems auf einem anorganischen Träger. Die Verwendung von Raney-Nickel ist nicht vorteilhaft, da es unter sauren Bedingungen nicht stabil ist. Das Nickel wird aus der Legierung herausgelöst und formt zusammen mit dem Säureanion das entsprechende Nickelsalz. Somit ist Raney-Nickel von den geeigneten erfindungsgemäßen Katalysatoren ausgeschlossen.

Im erfindungsgemäßen Verfahren wird der Katalysator, bezogen auf das eingesetzte 1,1-Difluor-2-nitroethan, in einer Konzentration von etwa 0,01 bis etwa 30 Gew.-% verwendet. Bevorzugt wird der Katalysator in einer Konzentration von etwa 0,1 bis etwa 25 Gew.-% verwendet.

Im erfindungsgemäßen Verfahren wird gewöhnlicherweise in einem ersten Schritt (i) 1,1-Difluor-2-nitroethan und der Katalysator vorgelegt und in einem zweiten Schritt (ii) Wasserstoff eingeleitet oder erzeugt. Die Umkehrung der Schritte (i) und (ii) ist möglich. Auch kann kontinuierlich oder diskontinuierlich (schub- bzw. batchweise) hydriert werden.

Die katalytische Hydrierung kann unter Überdruck (d.h. bis zu etwa 200 bar) in einem Autoklaven oder bei Normaldruck in einer Wasserstoffgasatmosphäre durchgeführt werden. Insbesondere bei hohen Reaktionstemperaturen kann es hilfreich sein, bei erhöhtem Druck zu arbeiten. Die (zusätzliche) Druckerhöhung kann durch Zuleiten eines Inertgases, wie Stickstoff oder Argon, bewirkt werden. Die erfindungsgemäße Hydrierung erfolgt bevorzugt bei einem Druck im Bereich von etwa 1 bis etwa 30 bar, besonders bevorzugt bei einem Druck im Bereich von etwa 5 bis etwa 25 bar.

In einer Ausführungsform der Erfindung wird eine ausreichende Menge an anorganischer oder organischer Säure oder Mischungen davon nach Hydrierung und gegebenenfalls nach Entfernen des Katalysators zugegeben. Geeignete Säuren sind beispielsweise Salzsäure, Schwefelsäure, Ameisen- oder Essigsäure. Hierdurch kann das entsprechende Säureadditionssalz von 2,2-Difluorethylamin erhalten, das dann wie hier beschrieben isoliert und/oder zum freien 2,2-Difluorethylamin umgesetzt wird.

Die katalytische Hydrierung findet in Gegenwart einer ausreichenden Menge an anorganischen oder organischen Säuren oder Mischungen davon statt. Geeignete Säuren sind beispielsweise Salzsäure, Schwefelsäure, Ameisen- oder Essigsäure. In dieser Ausführungsform darf der Katalysator nicht säureempfindlich sein. Bevorzugte Hydrierkatalysatoren sind deshalb insbesondere Platin und/oder Palladium enthaltende Katalysatoren. Die Säure kann in konzentrierter Form oder mit Wasser zugegeben werden. Vorzugsweise wird die Säure vor Hydrierung in die Reaktionsmischung gegeben, sodass im Idealfall ein Lösungsmittel-Säure Gemisch vorliegt. Das Vorhandensein der Säure führt dazu, dass das durch Hydrierung entstehende Produkt sofort zum entsprechenden Säureadditionssalz von 2,2-Difluorethylamin umgesetzt wird, wodurch weniger Nebenprodukte entstehen, was die Ausbeute erhöht. Auch führen Reaktionszeiten von weniger als 20 Stunden bereits zu guten Ausbeuten. Das entstandene Säureadditionssalz wird dann wie hier beschrieben isoliert und/oder zum freien 2,2-Difluorethylamin umgesetzt.

Unter ausreichender Menge an anorganischen oder organischen Säuren oder Mischungen davon, wird erfindungsgemäß die Menge verstanden, bei dem das Produkt 2,2-Difluorethylamin vollständig protoniert vorliegt. Bei einbasigen Säuren (z.B. HCl) benötigt man mindestens 1 Äquivalent an Säure und bei zweibasigen Säuren (wie H₂SO₄) mindestens 0,5 Äquivalent bezogen auf das eingesetzte 1,1-Difluor-2-nitroethan.

Es ist im Allgemeinen vorteilhaft, das erfindungsgemäße Verfahren in Gegenwart von Lösungsmitteln (Verdünnungsmitteln) durchzuführen. Die katalytische Hydrierung kann jedoch auch ohne ein Lösungsmittel durchgeführt werden. Lösungsmittel werden vorteilhafterweise in einer solchen Menge eingesetzt, dass das Reaktionsgemisch während des ganzen Verfahrens gut rührbar bleibt. Vorteilhafterweise wird, bezogen auf das eingesetzte 1,1-Difluor-2-nitroethan, die 1 bis 50-fache Lösungsmittelmenge, bevorzugt die 2 bis 40-fache Lösungsmittelmenge, besonders bevorzugt die 2 bis 30-fache Lösungsmittelmenge verwendet.

Als Lösungsmittel zur Durchführung des erfindungsgemäßen Verfahrens kommen alle unter den Reaktionsbedingungen inerten organischen Lösungsmittel in Frage, wobei die Art des verwendeten Lösungsmittels von der Art der Reaktionsdurchführung, insbesondere von der Art des verwendeten Katalysators und/oder der Wasserstoffquelle (Einleiten von gasförmigen Wasserstoff oder *in situ* Erzeugung) abhängt. Unter Lösungsmittel werden erfindungsgemäß auch Gemische reiner Lösungsmittel verstanden.

Erfindungsgemäß geeignete Lösungsmittel sind insbesondere Halogenkohlenwasserstoffe, z.B. Chlorkohlenwasserstoffe, wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol; Alkohole wie Methanol, Ethanol, Isopropanol, Butanol; Ether, wie Ethylpropylether, Methyl-tert-butylether, *n*-Butylether, Anisol, Phenetol, Cyclohexylmethylether, Dimethylether, Diethylether, Dimethylglycol Diphenylether, Diproplether, Diisopropylether, Di-*n*-butylether, Diisobutylether, Diisoamylether, Ethylenglycoldimethylether, Isopropylethylether, Methyl-tert-butylether, Tetrahydrofuran, Dioxan, Dichlordiethylether und Polyether des Ethylenoxids und/oder Propylenoxids; aliphatische, cycloaliphatische oder aromatische Kohlenwasserstoffe wie Pentan, Hexan, Heptan, Oktan, Nonan und technische Kohlenwasserstoffe welche durch Fluor- und Chloratome substituiert sein können, wie Methylenchlorid, Dichlormethan, Trichlormethan, Tetrachlorkohlenstoff, Fluorbenzol, Chlorbenzol oder Dichlorbenzol; beispielsweise sogenannte White Spirits mit Komponenten mit Siedepunkten im Bereich beispielsweise von 40 °C bis 250 °C, Cymol, Benzinfraktionen innerhalb eines Siedeinterwalles von 70 °C bis 190 °C, Cyclohexan, Methylcyclohexan, Petrolether, Ligroin, Octan, Benzol, Toluol, Chlorbenzol, Brombenzol, Xylol; Ester wie Methyl-, Ethyl-, Butyl-, Isobutylacetat, sowie Dimethyl-, Dibutyl- oder Ethylencarbonat. Erfindungsgemäße Lösungsmittel ist ferner Wasser.

Im erfindungsgemäßen Verfahren werden als Lösungsmittel bevorzugt Chlorkohlenwasserstoffe oder Alkohole verwendet.

Das erfindungsgemäße Verfahren kann in einem weiten Temperaturbereich (z.B. im Bereich von etwa -20 °C bis etwa 100 °C) durchgeführt werden. Vorzugsweise wird die katalytische Hydrierung in einem Temperaturbereich von etwa 0 °C bis etwa 40 °C durchgeführt.

Die Reaktionsdauer der erfindungsgemäßen Hydrierung liegt im Allgemeinen zwischen 30 Minuten und 24 Stunden. Längere Reaktionszeiten sind möglich und wirken sich auch nicht nachteilig aus, sind aber aus wirtschaftlichen Gesichtspunkten nicht vorteilhaft.

Liegt nach der katalytischen Hydrierung das 2,2-Difluorethylamin frei vor, so wird es, wenn nötig, durch Destillation gereinigt. Liegt 2,2-Difluorethylamin nach der katalytischen Hydrierung als Säureadditionssalz vor, dann erfolgt die Reinigung, wenn nötig, bevorzugt durch Kristallisation.

Wasserlösliche Säureadditionssalze von 2,2-Difluorethylamin werden im Allgemeinen durch Extraktion aus einer wässrigen Lösung gereinigt. Das freie 2,2-Difluorethylamin wird durch Umsetzen des entsprechenden Säureadditionssalzes mit organischen oder anorganischen Basen (z.B. NaHCO₃, Na₂CO₃ oder NaOH) freigesetzt. Anschließend wird das Difluorethylamin direkt aus der wässrigen Lösung abdestilliert oder in ein organisches Lösungsmittel extrahiert.

Die vorliegende Erfindung wird anhand der nachfolgenden Beispiele näher erläutert.

### Herstellungsbeispiele:

### Beispiel 1

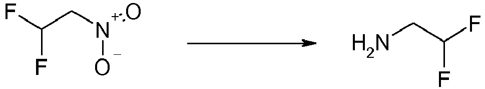

5 mmol 1,1-Difluor-2-nitroethan werden in Methanol vorgelegt und unter Stickstoff mit 20 Gew.-% PtO₂ versetzt. Das Reaktionsgemisch wird 16 Stunden bei Raumtemperatur (etwa 20 °C) und einem Druck von 20 bar Wasserstoff gerührt. Nach Abkühlen der Hydriermischung auf 0 °C wird der Druck abgelassen, d.h. entspannt, und der Katalysator abfiltriert. Danach werden 7,5 mmol HCl gelöst in Methanol zugegeben. Nach Entfernen des Lösungsmittels, hier Methanol, im Vakuum wird 560 mg 80 %-iges Hydrochlorid des 2,2-Difluorethylamins als Feststoff erhalten. Das entspricht einer Ausbeute von 80 % der Theorie.

NMR (MeOD): 6,19 bis 6,0 ppm (m, 1 H) 3,2 - 3,13 (m, 2H); M⁺: 81

### Beispiel 2

5 mmol 1,1-Difluor-2-nitroethan werden in Methanol zusammen mit 1,5 Equivalenten Salzsäure vorgelegt und unter Stickstoff mit 20 Gew.-% PtO₂ versetzt. Das Reaktionsgemisch wird 7 Stunden bei Raumtemperatur (etwa 20 °C) und einem Druck von 20 bar Wasserstoff gerührt. Nach Abkühlen der Hydriermischung auf 0 °C wird der Druck abgelassen, d.h. entspannt, und der Katalysator abfiltriert. Danach werden 7,5 mmol HCl gelöst in Methanol zugegeben. Nach Entfernen des Lösungsmittels, hier Methanol, im Vakuum wird 560 mg 95%-iges Hydrochlorid des 2,2-Difluorethylamins als Feststoff erhalten. Das entspricht einer Ausbeute von 95 % der Theorie.

NMR (MeOD): 6,19 bis 6,0 ppm (m, 1 H) 3,2 - 3,13 (m, 2H); M+: 81

### Beispiel 3:

In einem 25ml Hydrierautoklaven (Material: Hastelloy, mechanischer Propellerrührer) werden 1,0 g (0,007 mol, 1.0 Äquivalente) Difluornitroethan (ca. 80 %-ige Lösung in Dichlormethan) in 9 ml Ethanol mit 0,880 g (0,009 mol, 1,3 Äquivalente) konzentrierter Schwefelsäure und 0,100 g 20% Pd(OH)₂ auf Aktivkohle (Pearlman-Typ Katalysator) vorgelegt. Nach Spülen mit Wasserstoff wird ca. 3 Stunden bei Raumtemperatur und 20 bar Wasserstoff hydriert (Rührerdrehzahl ca. 600 U/min.). Es werden nach Filtration und nachwaschen des Katalysators 16,6 g einer schwach gelben Reaktionsmischung erhalten. Nach Analyse mittels quantitativer NMR ergibt sich eine Ausbeute von 90 %.

### Beispiel 4:

In einem 25ml Hydrierautoklaven (Material: Hasteloy, mechanischer Propellerrührer) werden 2,0 g (0,010 mol, 1.0 Äquivalente) Difluornitroethan (ca. 60 %-ige Lösung in Dichlormethan) in 9 ml Wasser mit 1,270 g (0,013 mol, 1,3 Äquivalente) konzentrierter Schwefelsäure und 0,100 g 20% Pd(OH)₂ auf Aktivkohle (Pearlman-Typ Katalysator) vorgelegt. Nach Spülen mit Wasserstoff wird ca. 3 Stunden bei Raumtemperatur und 20 bar Wasserstoff hydriert (Rührerdrehzahl ca. 600 U/min.). Es werden nach Filtration und Nachwaschen des Katalysators 17,8 g einer schwach gelben Reaktionsmischung erhalten. Nach Analyse mittels quantitativer NMR ergibt sich eine Ausbeute von 88 %.

### Vergleichsbeispiel 1:

5 mmol 1,1-Difluor-2-nitroethan werden in einem Gemisch von Methanol und konzentrierter Essigsäure (=Eisessig) im Verhältnis 4:1 vorgelegt und unter Stickstoff mit 20 Gew.-% Raney-Nickel versetzt. Das Reaktionsgemisch wird 7 Stunden bei Raumtemperatur (etwa 20 °C) und einem Druck von 20 bar Wasserstoff gerührt. Nach Abkühlen der Hydriermischung auf 0 °C wird der Druck abgelassen, d.h. entspannt, und der Katalysator abfiltriert. Danach wird das Lösungsmittels, hier Methanol, im Vakuum entfernt und man erhält 560 mg 95 %-iges 2,2-Difluorethylamin als Feststoff. Das entspricht einer Ausbeute von 80 % der Theorie. 2,2-Difluorethylamin liegt hier als Gemisch aus Monoacetyl-2,2-Difluorethylamin und seinem Acetatsalz vor. Der Raney-Nickel Katalysator kann nicht vollständig wiedergewonnen werden, da Nickel mit der Essigsäure zu Nickelacetat reagiert.
¹³C-NMR (MeOD) Monoacetyl-2,2-Difluorethylamin: -CHF₂ (115,3 ppm); -CH₂ (42,7 ppm), CO₂-(173,9 ppm); -CH₃ (22,3 ppm)
¹³C- NMR (MeOD) 2,2-Difluorethylamin als Acetatsalz: -CHF₂ (115,4 ppm); -CH₂ (43 ppm),CO₂-(176,5 ppm); -CH₃ (21,7 ppm)

### Vergleichsbeispiel 2:

11,1 g (0,1 mol Difluornitroethan, 0,1 g Eisenchlorid, 23 g Eisenspänen werden in einer Mischung von 50 ml konzentrierter Salzsäure und 80 ml Wasser 2 h unter Rückfluß erhitzt. Die Reaktionsmischung wird anschließend mit 32 g NaOH in 100 ml Wasser versetzt. Nach Wasserdampfdestillation wird das Destillat mit Salzsäure angesäuert und im Vakuum eingeengt. Es konnte kein Difluorethylaminhydrochlorid isoliert werden.

## Patentansprüche

1. Verfahren zur Herstellung von 2,2-Difluorethylamin, **dadurch gekennzeichnet dass** 1,1-Difluor-2-nitroethan einer katalytischen Hydrierung unterworfen wird, wobei der in der katalytischen Hydrierung eingesetzte Katalysator ein oder mehrere Metalle ausgewählt unter Palladium und Platin enthält, wobei Raney-Nickel ausgeschlossen ist, wobei die katalytische Hydrierung in Gegenwart von anorganischer oder organischer Säure stattfindet und wobei der Katalysator PtO₂ oder Pd(OH)₂ auf Aktivkohle ist.

2. Verfahren nach Anspruch 1, wobei bei der katalytischen Hydrierung gasförmiger Wasserstoff in das Reaktionsgefäß eingeleitet oder *in situ* erzeugt wird.

## Claims

1. Process for preparing 2,2-difluoroethylamine, **characterized in that** 1,1-difluoro-2-nitroethane is subjected to a catalytic hydrogenation, wherein the catalyst used in the catalytic hydrogenation comprises one or more metals selected from palladium and platinum, wherein Raney nickel is excluded, wherein the catalytic hydrogenation takes place in the presence of inorganic or organic acid and wherein the catalyst is PtO₂ or Pd(OH)₂ on activated carbon.

2. Process according to Claim 1, wherein the catalytic hydrogenation involves introducing gaseous hydrogen into the reaction vessel or generating it in situ.

## Revendications

1. Procédé de préparation de 2,2-difluoroéthylamine, **caractérisé en ce que** du 1,1-difluoro-2-nitroéthane est soumis à une hydratation catalytique, le catalyseur utilisé dans l'hydratation catalytique contenant un ou plusieurs métaux choisis parmi le palladium et le platine, le nickel de Raney étant exclu, l'hydratation catalytique ayant lieu en présence d'acide inorganique ou organique et le catalyseur étant du PtO₂ ou du Pd(OH)₂ sur du charbon actif.

2. Procédé selon la revendication 1, dans lequel, lors de l'hydratation catalytique, de l'hydrogène gazeux est introduit dans le récipient réactionnel ou est généré *in situ.*
